# EUROPEAN PATENT APPLICATION

(11) **EP 4 275 687 A1**
(43) Date of publication of application: **15.11.2023**
(21) Application number: 22742844.8
(22) Date of filing: 20.01.2022
(51) Int. Cl.: A61K 31/5377, A61K 9/00, A61P 3/04

(54) **USES OF MELANOCORTIN-4 RECEPTOR AGONIST**

(30) Priority: 21.01.2021 KR 20210008622
(71) Applicant: Lg Chem, Ltd., Seoul 07336 (KR)
(72) Inventor: PARK, Hee Dong, Seoul 07796 (KR); YEO, Su Jin, Seoul 07796 (KR); AHN, Hye Won, Seoul 07796 (KR)
(74) Representative: Hoffmann Eitle
(86) International application number: PCT/KR2022/001021
(87) International publication number: WO 2022/158868

(57) **Abstract**

The present invention relates to uses of a compound of chemical formula 1 or a pharmaceutically acceptable salt thereof for the purpose of preventing or treating rare genetic obesity diseases, particularly rare genetic obesity diseases associated with a damaged melanocortin-4 receptor (MC4R) pathway.

## Description

### TECHNICAL FIELD

The present invention relates to the use of a compound of the following Formula 1 or a pharmaceutically acceptable salt thereof in the prevention or treatment of rare genetic obesities, specifically rare genetic obesity associated with impaired melanocortin-4 receptor (MC4R) pathway: 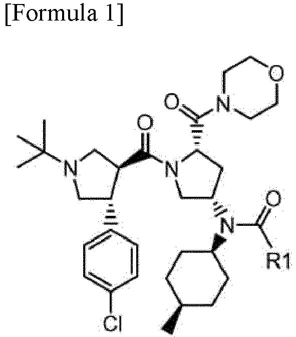 wherein R1 is C₂-C₅ alkyl.

### BACKGROUND ART

Melanocortin receptor (MCR) is a type of G-protein coupled receptor (GPCR), and the main role of G-protein is to activate secondary messengers and regulate the response of cells to many physiological stimuli through signal transduction. Until now, five subtypes of melanocortin receptor have been identified. MC1R is mainly expressed in melanocytes and macrophages, and determines the color of skin and hair by regulating melanin pigment in melanocytes. MC2R is expressed in the adrenal gland and adipose tissue, and the mediating function of adrenal hormone secretion regulation by adrenocorticotropic hormone in the adrenal gland is well known. MC3R, MC4R and MC5R are expressed not only in nerve terminals but also in the brain, and thus it is understood that they mediate central nerve actions by melanocortin peptides, which are expressed as effects on behavior, learning, memory, appetite, and generation and regeneration of nerves. Until now, MC3R is known to be involved in erectile dysfunction and inflammatory response, and MC4R is known to be involved in obesity and diabetes, and studies on the specificity of actions of each receptor are being actively carried out (MacNeil DJ et al., Eur J Pharmacol 2002, 450, 93). As a result, it was found that MC4R is deeply involved in genetic studies in people with obesity, and it was demonstrated that this receptor plays an important role in appetite regulation by showing that knockout mice in which MC4R was removed develop obesity by overeating (Lu D, Willard D et al., Nature 1994, 371(6500), 799; Huszar D et al., Cell 1997, 88(1), 131; Hinney A et al., J Clin Endocrinol Metab 1990, 84(4), 1483).

Meanwhile, as previously developed anti-obesity drugs, appetite suppressants acting on the central nervous system are the main types. Among them, drugs that control the action of neurotransmitters (for example, phentermine, mazindol, lorcaserin, fluoxetine and sibutramine) were mostly used. However, the neurotransmitter modulators exert a wide range of effects on various physiological actions in addition to appetite suppression by numerous subtype receptors. Therefore, the modulators are lacking in selectivity for each subtype and have a large disadvantage in that various side effects are accompanied in case of long-term administration. On the other hand, melanocortin agonists are neuropeptides, not neurotransmitters, and considering that all other functions other than energy metabolism are normal in the MC4R gene knockout (KO) mice, melanocortin agonists have an advantage as an action point in that only weight loss through appetite suppression can be induced without affecting other physiological functions.

### DISCLOSURE OF INVENTION

### TECHNICAL PROBLEM

The present invention is intended to provide the use of a compound of the following Formula 1 or a pharmaceutically acceptable salt thereof in the prevention or treatment of rare genetic obesities, specifically rare genetic obesity associated with impaired melanocortin-4 receptor (MC4R) pathway: 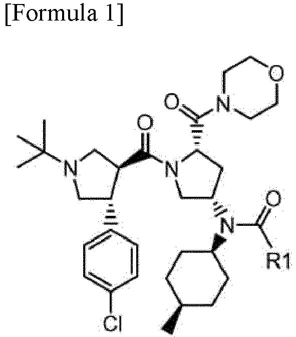 wherein R1 is C₂-C₅ alkyl.

### SOLUTION TO PROBLEM

The present invention provides a medicament for the prevention or treatment of rare genetic obesity, which comprises a therapeutically effective amount of a compound of the following Formula 1 or a pharmaceutically acceptable salt thereof: 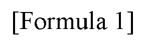 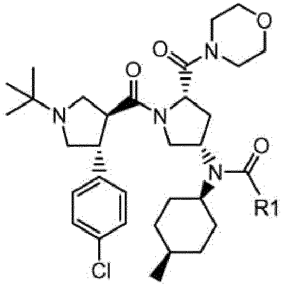 wherein R1 is C₂-C₅ alkyl.

In addition, the present invention provides a pharmaceutical composition for the prevention or treatment of rare genetic obesity, which comprises a therapeutically effective amount of the compound of Formula 1 or a pharmaceutically acceptable salt thereof, together with a pharmaceutically acceptable carrier.

In addition, the present invention provides a method for the prevention or treatment of rare genetic obesity, which comprises administering a therapeutically effective amount of the compound of Formula 1 or a pharmaceutically acceptable salt thereof to a subject in need thereof.

In addition, the present invention provides use of the compound of Formula 1 or a pharmaceutically acceptable salt thereof in the prevention or treatment of rare genetic obesity.

The present invention is described in detail hereinafter.

According to one aspect of the present invention, there is provided a medicament for the prevention or treatment of rare genetic obesity, which comprises a therapeutically effective amount of the compound of Formula 1 or a pharmaceutically acceptable salt thereof.

According to another aspect of the present invention, there is provided a pharmaceutical composition for the prevention or treatment of rare genetic obesity, which comprises a therapeutically effective amount of the compound of Formula 1 or a pharmaceutically acceptable salt thereof, together with a pharmaceutically acceptable carrier.

In one embodiment according to the present invention, the compound of Formula 1 is *N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N*-((1*s*,4*R*)-4-methylcyclohexyl)isobutyramide of the following Formula 2:

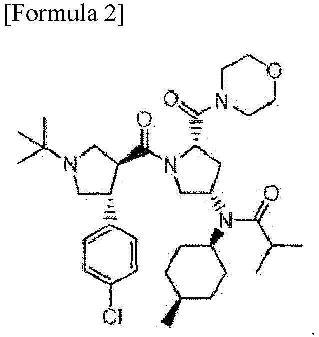

In another embodiment according to the present invention, examples of the pharmaceutically acceptable salt include acid addition salts formed by an inorganic acid such as hydrochloric acid, sulfuric acid, nitric acid, phosphoric acid, hydrobromic acid, and hydroiodic acid; an organic carboxylic acid such as tartaric acid, formic acid, citric acid, acetic acid, trichloroacetic acid, trifluoroacetic acid, gluconic acid, benzoic acid, lactic acid, fumaric acid, and maleic acid; and sulfonic acid such as methanesulfonic acid, benzenesulfonic acid, p-toluenesulfonic acid, and naphthalenesulfonic acid, but is not limited thereto. In another embodiment according to the present invention, the pharmaceutically acceptable salt may be selected from the group consisting of hydrochloric acid, sulfuric acid, nitric acid, phosphoric acid, hydrobromic acid and hydroiodic acid. In another embodiment according to the present invention, the pharmaceutically acceptable salt is hydrochloride.

In another embodiment according to the present invention, the hydrochloride of the compound of Formula 1 may be prepared according to the following Reaction Scheme 1. However, a person skilled in the art may prepare the compound of Formula 1 by various methods based on the structure of Formula 1. 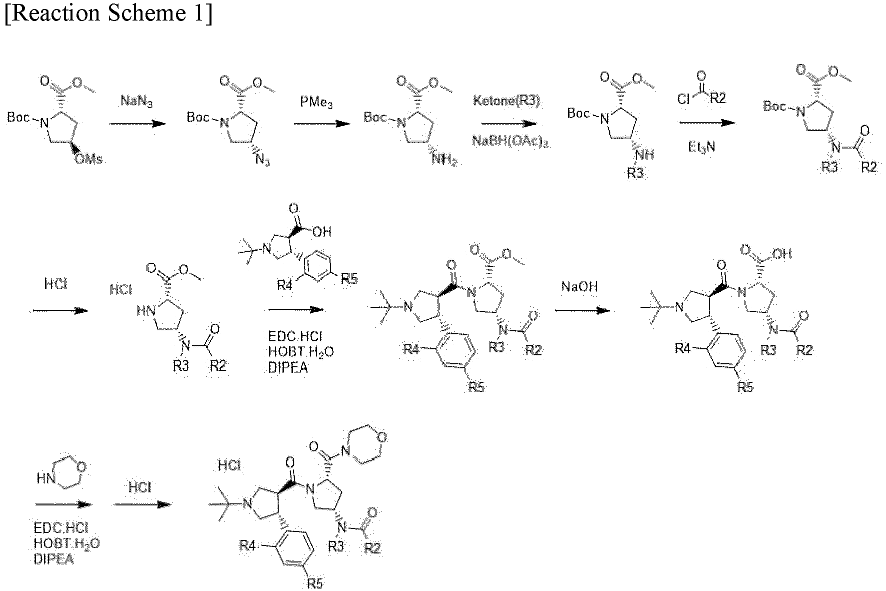 In Reaction Scheme 1,
R2 is C₁-C₅ alkyl;
R3 is C₃-C₈ cycloalkyl unsubstituted or substituted with 1 or 2 C₁-C₅ alkyl; and
R4 and R5 are each independently hydrogen or halogen.

In another embodiment according to the present invention, the rare genetic obesity may be associated with impaired melanocortin-4 receptor (MC4R) pathway. In another embodiment according to the present invention, the rare genetic obesity associated with impaired melanocortin-4 receptor (MC4R) pathway may be leptin receptor (LEPR) deficiency. Leptin is a hormone secreted by body fat cells (adipocytes). Leptin hormone acts on leptin receptors in the hypothalamus to maintain metabolism and appetite homeostasis at the upstream of the melanocortin-4 receptor (MC4R) pathway and plays an important role in regulating body weight. Thus, leptin receptor deficiency can lead to severe obesity.

In another embodiment according to the present invention, a "therapeutically effective amount" for an individual subject refers to an amount sufficient to achieve the above pharmacological effect-i.e., the therapeutic effect as described above. The amount of the compound may vary depending on the condition and severity of the subject, the mode of administration and the age of the subject to be treated, but could be determined by persons of ordinary skill in the art based on their knowledge.

In another embodiment according to the present invention, the therapeutically effective dosage of the compound of Formula 1 is, for example, typically in the range of about 0.1 to 500 mg per day according to the frequency and intensity of administration. A typical daily dose of intramuscular or intravenous administration for adults is in the range of about 0.1 to 300 mg per day which can be administered in divided unit dosages. Some patients may need a higher daily dose.

In the present invention, a "pharmaceutical composition" may include other components such as carriers, diluents, excipients, etc., in addition to the active ingredient of the present invention. Accordingly, the pharmaceutical composition may include pharmaceutically acceptable carriers, diluents, excipients or combinations thereof, if necessary. The pharmaceutical composition facilitates the administration of compounds into the body. Various methods for administering the compounds include, but are not limited to, oral, injection, aerosol, parenteral and local administration.

Herein, a "carrier" means a compound that facilitates the addition of compounds into the cell or tissue. For example, dimethylsulfoxide (DMSO) is a conventional carrier facilitating the administration of many organic compounds into living cells or tissues.

Herein, a "diluent" means a compound that not only stabilizes a biologically active form but is diluted in solvent dissolving the compounds. A dissolved salt in buffer is used as a diluent in this field. A conventionally used buffer is a phosphate buffer saline mimicking salt form in body fluid. Since a buffer solution can control the pH of the solution at low concentration, a buffer diluent hardly modifies the biological activity of compounds.

Herein, "pharmaceutically acceptable" means such property that does not impair the biological activity and physical property of compounds.

The compounds according to the present invention can be formulated as various pharmaceutically administered dosage forms. In the preparation of the pharmaceutical composition of the present invention, an active component-specifically, the compound of Formula 1 or a pharmaceutically acceptable salt thereof-is mixed with selected pharmaceutically acceptable carriers considering the dosage form to be prepared. For example, the pharmaceutical composition of the present invention can be formulated as injections, oral preparations and the like, as needed.

The compound of the present invention can be formulated by conventional methods using known pharmaceutical carriers and excipients, and inserted into a unit or multi-unit containers. The formulations may be solution, suspension or emulsion in oil or aqueous solvent and include conventional dispersing agents, suspending agents or stabilizing agents. In addition, the compound may be, for example, dry powder form which is dissolved in sterilized pyrogen-free water before use. The compound of the present invention can be formulated into suppositories by using a conventional suppository base such as cocoa butter or other glycerides. Solid forms for oral administration include capsules, tablets, pills, powders and granules. Capsules and tablets are preferred. Tablets and pills are preferably enteric-coated. Solid forms are manufactured by mixing the compounds of the present invention with at least one carrier selected from inert diluents such as sucrose, lactose or starch, lubricants such as magnesium stearate, disintegrating agents, binders and the like. In addition, it may be formulated as a transdermal dosage form-for example, as a lotion, ointment, gel, cream, patch or spray.

Herein, the term "prevention" refers to reducing or eliminating the possibility of contracting a disease.

Herein, the term "treatment" is used to mean deterring, delaying or ameliorating the progress of diseases in a subject exhibiting symptoms of diseases.

### EFFECTS OF THE INVENTION

The medicament or pharmaceutical composition according to the present invention can effectively prevent or treat rare genetic obesities, specifically rare genetic obesity associated with impaired melanocortin-4 receptor (MC4R) pathway.

### BRIEF DESCRIPTION OF DRAWINGS

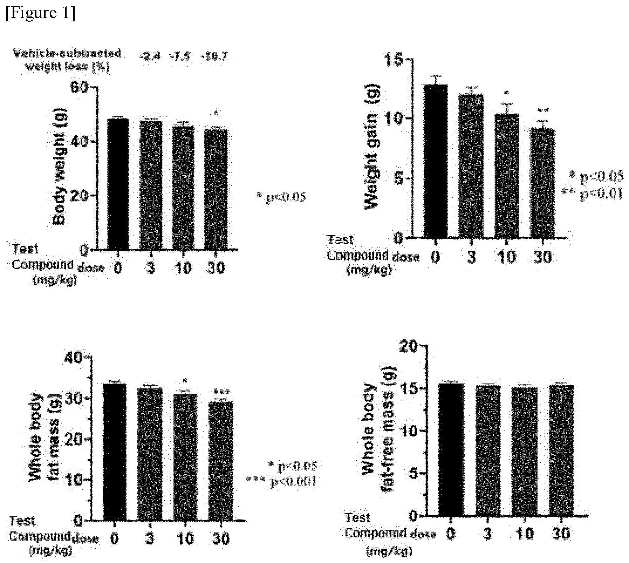

Figure 1 is graphs representing the results of measuring the weight loss effect in a leptin receptor-deficient mouse model (db/db mouse model).

### MODE FOR THE INVENTION

Hereinafter, the present invention is explained in more detail with the following examples. However, it must be understood that the protection scope of the present invention is not limited to the examples.

### Preparation Example: Synthesis of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

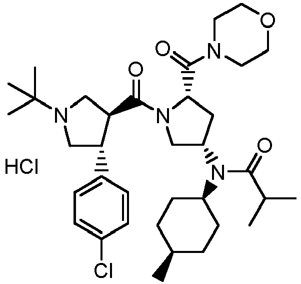

The title compound was obtained through Steps A, B, C and D.

### Step A: Preparation of methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate

Methyl (2*S*,4*S*)-4-(*N*-((1*s*,4*R*)-4-methylcyclohexyl)isobutyramido)pyrrolidine-2-carboxylate hydrochloride (28.7 g, 82.73 mmol), (3*S*,4*R*)-1-(*tert*-butyl)-4-(4-chlorophenyl)pyrrolidine-3-carboxylic acid (24.5 g, 86.87 mmol), 1-(3-dimethylaminopropyl)-3-ethylcarbodiimide hydrochloride (22.2 g, 115.83 mmol), and 1-hydroxybenzotriazole hydrate (15.7 g, 115.83 mmol) were dissolved in *N*,*N*'-dimethylformamide (400 mL), and *N*,*N*'-diisopropylethylamine (72.0 mL, 413.66 mmol) was slowly added. After stirring at room temperature for 16 hours, the reaction solvent was concentrated under reduced pressure, then a 0.5 N aqueous sodium hydroxide solution was added, and extraction was performed twice with ethyl acetate. The organic layer was washed twice with an aqueous sodium chloride solution and water, dried over anhydrous magnesium sulfate, and filtered. The filtrate was concentrated under reduced pressure and purified by column chromatography to obtain the title compound (41.19 g, 87%).
MS [M+H] = 575 (M+1)

### Step B: Preparation of (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylic acid

Methyl (2*S*,4*S*)-1-((3*S*,4*R*)-1-(*tert*-butyl)-4-(4-chlorophenyl)pyrrolidine-3-carbonyl)-4-(*N*-((1*s*,4*R*)-4-methylcyclohexyl)isobutyramido)pyrrolidine-2-carboxylate (39.4 g, 68.62 mmol) obtained in Step A was dissolved in methanol (450 mL), and a 6N aqueous sodium hydroxide solution (57.2 mL, 343.09 mmol) was added. After stirring at room temperature for 16 hours and adjusting pH to about 5 with a 6 N aqueous hydrochloric acid solution, the reaction solution was concentrated under reduced pressure. The concentrate was dissolved in dichloromethane, and then the insoluble solid was filtered with a paper filter. The filtrate was concentrated under reduced pressure to obtain crude (38.4 g, 99%), which was used in the next step without purification.
MS [M+H] = 561 (M+1)

### Step C: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide

(2*S*,4*S*)-1-((3*S*,4*R*)-1-(*tert*-butyl)-4-(4-chlorophenyl)pyrrolidine-3-carbonyl)-4-(*N-*((1*s*,4*R*)-4-methylcyclohexyl)isobutyramido)pyrrolidine-2-carboxylic acid (38.4 g, 68.60 mmol) obtained in Step B, 1-(3-dimethylaminopropyl)-3-ethylcarbodiimide hydrochloride (18.4 g, 96.04 mmol), and 1-hydroxybenzotriazole hydrate (13.0 g, 96.04 mmol) were dissolved in *N*,*N*'-dimethylformamide (200 mL), and morpholine (5.9 mL, 68.80 mmol) and *N,N'-*diisopropylethylamine (59.7 mL, 343.02 mmol) were slowly and sequentially added. After stirring at room temperature for 16 hours, the reaction solution was concentrated under reduced pressure, a 0.5 N aqueous sodium hydroxide solution was added, and extraction was performed twice with ethyl acetate. The organic layer was washed twice with an aqueous sodium chloride solution and water, dried over anhydrous magnesium sulfate, and filtered. The filtrate was concentrated under reduced pressure and purified by column chromatography to obtain the title compound (37.05 g, 86%).
MS [M+H] = 630 (M+1)

### Step D: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

*N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N*-((1*s*,4*R*)-4-methylcyclohexyl)isobutyramide (5.0 g, 7.95 mmol) obtained in Step C was dissolved in ethyl acetate (50 mL), and a 2N hydrochloric acid ethyl acetate solution (3.97 mL, 15.89 mmol) was slowly added. After stirring at room temperature for 30 minutes, the reaction solvent was concentrated under reduced pressure. The resulting crude solid was purified by trituration using hexane and diethyl ether to obtain the title compound (5.23 g, 99%).
MS [M+H] = 630 (M+1)
¹H NMR (500 MHz, CD₃OD) δ 7.49-7.44 (m, 4H), 4.83 (m, 1H), 4.23-4.20 (m, 1H), 3.95-3.91 (m, 2H), 3.79-3.47 (m, 14H), 3.03-3.00 (m, 1H), 2.86-2.82 (m, 1H), 2.73-2.67 (m, 1H), 2.20-2.14 (m, 1H), 1.97 (m, 1H), 1.80-1.62 (m, 5H), 1.50 (s, 9H), 1.44-1.27 (m, 3H), 1.06-1.04 (m, 9H)

### Example: db/db mouse model experiment

The db/db mouse having a mutation in the leptin receptor gene that acts on the upstream of the melanocortin-4 receptor (MC4R) is a model that reflects leptin receptor deficiency obesity, and this mouse exhibits abnormal metabolic disease-related phenotypes such as hyperphagia and severe obesity. To confirm anti-obesity efficacy, *N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride (hereinafter referred to as "Test Compound") obtained in the Preparation Example was administered repeatedly for 4 weeks while feeding 45 kcal% high-fat diet (diet with 45 kcal% fat) to db/db mice which is a genetic obesity mouse model in which a mutation in the leptin receptor associated with the MC4R pathway occurred. The weight gain inhibitory effect was confirmed by measuring the body weight during the administration period, and the body fat mass reduction effect was confirmed by measuring the total body fat mass after repeated administration for 4 weeks. As a result, the body weight was significantly reduced compared to the negative control group, and the weight gain rate was also significantly decreased. As a result of body fat analysis, the fat-free mass showed no difference compared to the negative control group, whereas the fat mass was significantly decreased compared to the negative control group. The results are represented in Figure 1.

As can be seen from Figure 1, it was confirmed that the Test Compound according to the present invention shows a significant weight loss effect.

## Claims

1. A medicament for the prevention or treatment of rare genetic obesity, which comprises a therapeutically effective amount of a compound of the following Formula 1 or a pharmaceutically acceptable salt thereof: 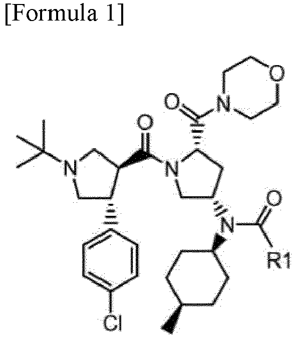 wherein R1 is C₂-C₅ alkyl.

2. The medicament according to Claim 1, wherein the compound of Formula 1 is *N-*((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N*-((1*s*,4*R*)-4-methylcyclohexyl)isobutyramide of the following Formula 2:

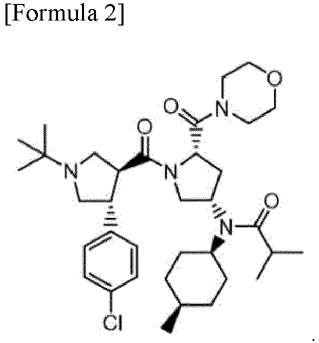

3. The medicament according to Claim 1, wherein the pharmaceutically acceptable salt is selected from the group consisting of hydrochloric acid, sulfuric acid, nitric acid, phosphoric acid, hydrobromic acid and hydroiodic acid.

4. The medicament according to Claim 1, wherein the rare genetic obesity is associated with impaired melanocortin-4 receptor (MC4R) pathway.

5. The medicament according to Claim 4, wherein the rare genetic obesity associated with impaired melanocortin-4 receptor (MC4R) pathway is leptin receptor (LEPR) deficiency.

6. The medicament according to Claim 1, which is an oral formulation.

7. A pharmaceutical composition for the prevention or treatment of rare genetic obesity, which comprises a therapeutically effective amount of a compound of the following Formula 1 or a pharmaceutically acceptable salt thereof, together with a pharmaceutically acceptable carrier: 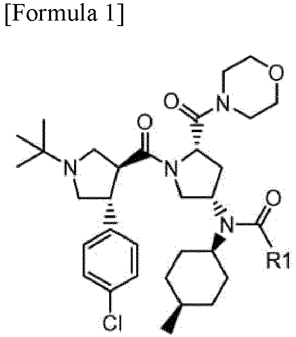 wherein R1 is C₂-C₅ alkyl.

8. The pharmaceutical composition according to Claim 7, wherein the compound of Formula 1 is *N*-((3*S*,5*S*)-1-((3*S*,4R)-1-(*tert*-butyl)-4-(4-chlorophenyl)pyrrolidine-3-carbonyl)-5-(morpholine-4-carbonyl)pyrrolidin-3-yl)-*N*-((1*s*,4*R*)-4-methylcyclohexyl)isobutyramide of the following Formula 2:

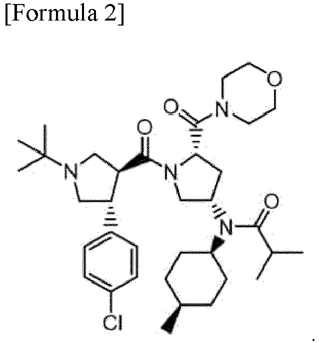

9. The pharmaceutical composition according to Claim 7, wherein the pharmaceutically acceptable salt is selected from the group consisting of hydrochloric acid, sulfuric acid, nitric acid, phosphoric acid, hydrobromic acid and hydroiodic acid.

10. The pharmaceutical composition according to Claim 7, wherein the rare genetic obesity is associated with impaired melanocortin-4 receptor (MC4R) pathway.

11. The pharmaceutical composition according to Claim 10, wherein the rare genetic obesity associated with impaired melanocortin-4 receptor (MC4R) pathway is leptin receptor (LEPR) deficiency.

12. The pharmaceutical composition according to Claim 7, which is an oral formulation.
